# EUROPÄISCHE PATENTSCHRIFT

(11) **EP 2 320 970 B1**
(45) Veröffentlichungstag und Bekanntmachung des Hinweises auf die Patenterteilung: **15.05.2013**
(21) Anmeldenummer: 09777890.6
(22) Anmeldetag: 14.08.2009
(51) Int. Cl.: A61M 1/16, F04B 43/12

(54) **Verfahren und Vorrichtung zum Überwachen einer peristaltischen Schlauchpumpe zur Förderung einer Flüssigkeit in einer Schlauchleitung**
Method and apparatus for monitoring a peristaltic pump for conducting a fluid in a tubing
Procédé et dispositif de surveillance d'une pompe péristaltique pour le refoulement d'un liquide dans un tuyau flexible

(30) Priorität: 21.08.2008 DE 102008039022
(43) Veröffentlichungstag der Anmeldung: 18.05.2011
(73) Patentinhaber: Fresenius Medical Care Deutschland GmbH, 61352 Bad Homburg v.d.H. (DE)
(72) Erfinder: KOPPERSCHMIDT, Pascal, 97456 Dittelbrunn (DE); SPICKERMANN, Reiner, 97535 Wasserlosen-Burghausen (DE)
(74) Vertreter: Oppermann, Frank
(86) Internationale Anmeldenummer: PCT/EP2009/005915
(87) Internationale Veröffentlichungsnummer: WO 2010/020380

(56) Entgegenhaltungen:
- WO-A1-97/45150
- WO-A2-2007/104435
- DE-A1- 10 305 036
- DE-U1-202007 015 453
- US-A1- 2001 021 817

## Beschreibung

Die Erfindung betrifft ein Verfahren zum Überwachen einer peristaltischen Schlauchpumpe zur Förderung einer Flüssigkeit in einer Schlauchleitung, insbesondere einer peristaltischen Schlauchpumpe einer extrakorporalen Blutbehandlungsvorrichtung, die mehrere Verdrängerkörper zum Okkludieren der Schlauchleitung aufweist. Darüber hinaus bezieht sich die Erfindung auf eine Vorrichtung zum Überwachen einer peristaltischen Schlauchpumpe, insbesondere einer peristaltischen Schlauchpumpe einer extrakorporalen Blutbehandlungsvorrichtung, sowie eine extrakorporale Blutbehandlungsvorrichtung mit einer Vorrichtung zur Überwachung einer peristaltischen Schlauchpumpe.

Bei einer extrakorporalen Blutbehandlung, beispielsweise einer Hämodialyse, durchströmt das zu behandelnde Blut in einem extrakorporalen Blutkreislauf die Blutkammer eines durch eine semipermeable Membran in die Blutkammer und eine Dialysierflüssigkeitskammer unterteilten Dialysators, während in einem Dialysierflüssigkeitssystem Dialysierflüssigkeit die Dialsysierflüssigkeitskammer des Dialysators durchströmt. Der extrakorporale Blutkreislauf weist eine arterielle Schlauchleitung auf, die zu der Blutkammer führt, und eine venöse Schlauchleitung auf, die von der Blutkammer abgeht. Die Schlauchleitungen der extrakorporalen Blutbehandlungsvorrichtung werden im Allgemeinen als zur einmaligen Verwendung bestimmte Disposable bereitgestellt. Die bekannten Blutbehandlungsvorrichtungen verfügen über eine Blutpumpe, die im Allgemeinen stromauf der Blutkammer des Dialysators angeordnet ist, um einen ausreichenden Blutfluss im extrakorporalen Blutkreislauf sicher zu stellen.

An die Blutpumpen werden hohe technische Anforderungen gestellt. Daher kommen nur bestimmte Pumpentypen in Betracht. In der Praxis haben sich Schlauchpumpen bewährt, die das Blut des Patienten durch die arterielle und venöse Schlauchleitung fördern.

Die Schlauchpumpen werden nach ihrer Arbeitsweise auch als peristaltische Pumpen bezeichnet. Ihre Pumpwirkung basiert darauf, dass sich mindestens eine Eng- oder Verschlussstelle (Okklusion) längs der als Pumpenraum dienenden elastischen Schlauchleitung bewegt und dadurch die eingeschlossene Flüssigkeit in Förderrichtung verschiebt.

Bei der gebräuchlichsten Bauart der Schlauchpumpen erfolgt die Einstellung so, dass der elastische Schlauch an den bewegten Engstellen vollständig verschlossen wird. Daher werden diese Pumpen auch als okklusive Schlauchpumpen bezeichnet.

Die beweglichen Eng- oder Verschlussstellen, die das Blut im Pumpenschlauch transportieren, können unterschiedlich ausgebildet sein. Es sind Rollenpumpen bekannt, bei denen der Schlauch zwischen einem Stator, der eine gebogene Rollenbahn als Widerlager bildet, und einem drehbar darin gelagerten, mit Rollen besetzten Rotor eingelegt wird, so dass sich die Rollen in Förderrichtung auf dem Schlauch abwälzen. Vorzugsweise sind die Rollen federnd an dem Rotor gelagert, so dass sie auf den Schlauch eine Anpresskraft ausüben. Daneben sind auch Fingerpumpen bekannt, bei denen die Verschlusskörper durch eine längs des Schlauchs angeordnete Reihe von beweglichen Stempeln (Fingern) gebildet werden.

Einen Überblick über die unterschiedlichen Bauarten der Rollen- und Fingerpumpen wird in Dialysetechnik, 4. Auflage, Gesellschaft für angewandte Medizintechnik m.b.H und Co. KG, Friedrichsdorf, 1988 gegeben.

An den ordnungsgemäßen Betrieb derartiger Schlauchpumpen bei der Verwendung in medizin-technischen Einrichtungen, insbesondere bei Blutbehandlungsvorrichtungen, werden hohe Anforderungen gestellt. Peristaltische Schlauchpumpen werden in den bekannten Blutbehandlungsvorrichtungen nicht nur zur Förderung des Bluts, sondern auch zur Förderung anderer Flüssigkeiten eingesetzt.

Während des Betriebs der peristaltischen Schlauchpumpe besteht die Gefahr, dass die in die Schlauchpumpe eingelegte Schlauchleitung nicht ordnungsgemäß im Pumpenbett liegt, oder die Verdrängerkörper nicht ordnungsgemäß über die Schlauchleitung geführt werden. Wenn die peristaltische Schlauchpumpe einen derartigen Defekt aufweist, ist ein ordnungsgemäßer Betrieb der Schlauchpumpe nicht sichergestellt. Bei einer nicht korrekten Lage des Schlauchsegments im Pumpenbett besteht insbesondere die Gefahr einer Beschädigung der Schlauchleitung. Auf jeden Fall ist aufgrund einer möglicherweise nicht ausreichenden Okklusion der Schlauchleitung eine ordnungsgemäße Förderung der Flüssigkeit nicht sichergestellt.

Die US-A-5,629,871 beschreibt ein Verfahren und eine Vorrichtung zur Überwachung der Funktionsfähigkeit einzelner Baugruppen einer Hämodialysevorrichtung. Zu diesen zählen auch die Schlauchpumpen, wobei der Pumpenstrom oder die Versorgungsspannung der Schlauchpumpen überwacht wird, um einen Ausfall der Pumpe feststellen zu können.

Aus der US-A-4,781,525 ist bekannt, den Pumpenstrom zur Bestimmung der Förderrate heranzuziehen. Die WO 97/45150 beschreibt ein Verfahren zur Bestimmung des Förderdrucks einer Pumpe, bei dem der Pumpenstrom bestimmt wird.

Aus der WO 2007/104435 A2 ist ein Verfahren und eine Vorrichtung zum Betreiben einer elektrischen peristaltischen Schlauchpumpe, insbesondere einer Schlauchpumpe zum Fördern von Flüssigkeiten in extrakorporalen Blutbehandlungsvorrichtungen bekannt. Zur Überwachung des ordnungsgemäßen Betriebs der Schlauchpumpe wird die Leistungsaufnahme der Pumpe oder eine mit der Leistungsaufnahme korrelierende physikalische Größe überwacht. Die Überwachung des Pumpenstroms beruht darauf, dass der Pumpenstrom einen sich periodisch nicht ändernden Gleichanteil aufweist, dem ein sich periodisch ändernder Wechselteil überlagert ist. Zur Überwachung des ordnungsgemäßen Betriebs der Schlauchpumpe wird während der Blutbehandlung überwacht, wie der Wechselanteil der Leistungsaufnahme im Verhältnis zum Gleichanteil der Leistungsaufnahme ansteigt bzw. abfällt.

Der Erfindung liegt die Aufgabe zugrunde, den ordnungsgemäßen Betrieb einer peristaltischen Schlauchpumpe zu überwachen, die mehrere Verdrängerkörper zum Okkludieren einer Schlauchleitung aufweist, insbesondere einer peristaltischen Schlauchpumpe einer extrakorporalen Blutbehandlungsvorrichtung.

Darüber hinaus liegt der Erfindung die Aufgabe zugrunde, eine Vorrichtung zum Überwachen des ordnungsgemäßen Betriebs einer peristaltischen Schlauchpumpe, insbesondere einer peristaltischen Schlauchpumpe einer extrakorporalen Blutbehandlungsvorrichtung bereit zu stellen.

Eine weitere Aufgabe der Erfindung ist eine extrakorporale Blutbehandlungsvorrichtung mit einer Vorrichtung zum Überwachen des ordnungsgemäßen Betriebs einer peristaltischen Schlauchpumpe zu schaffen.

Die Lösung dieser Aufgaben erfolgt erfindungsgemäß mit den Merkmalen der Patentansprüche 1, 9 und 15. Vorteilhafte Ausführungsformen der Erfindung sind Gegenstand der Unteransprüche.

Das erfindungsgemäße Verfahren und die erfindungsgemäße Vorrichtung beruhen darauf, dass während des Betriebs der Pumpe die von der Pumpe aufgenommene Leistung und/oder der Druck im Schlauchsegment stromauf oder stromab der Pumpe überwacht wird, wobei die Leistung bzw. der Druck in den jeweiligen Verdrängerkörpern zugeordneten Zeitbereichen erfasst wird, so dass den einzelnen Verdrängerkörpern zugeordnete Messsignale ermittelt werden. Dem erfindungsgemäßen Verfahren und der erfindungsgemäßen Vorrichtung liegen die Erkenntnis zugrunde, dass bei einem nicht ordnungsgemäßen Betrieb der Schlauchpumpe sich die Leistungs- bzw. Drucksignale, die den einzelnen Verdrängerkörpern zugeordnet werden können, deutlich voneinander unterscheiden. Bei einem ordnungsgemäßen Betrieb der Pumpe hingegen sind signifikante Unterschiede im Hinblick auf die Leistungsaufnahme oder den Druck im Schlauchsegment stromauf oder stromab der Pumpe nicht zu erkennen, da sämtliche Verdrängerkörper der Pumpe auf die Leistungsaufnahme oder den Druck den gleichen Einfluss haben. Besonders signifikant unterscheiden sich die Leistungsaufnahme und der Druck, wenn das Blutschlauchsegment nicht ordnungsgemäß im Pumpenbett liegt. Aber auch eine nicht ordnungsgemäße Führung der Verdrängerkörper führt zu derartigen Unsymmetrien. Bei Rollenpumpen beispielsweise können Defekte der Rollenlager oder der Rückstellfedern der einzelnen Rollen zu Unterschieden hinsichtlich der Leistungsaufnahme oder des Drucks führen.

In dem Augenblick, in dem die Verdrängerkörper, beispielsweise die Rollen der Rollenpumpe auf das Schlauchsegment der Schlauchleitung treffen, erhöht sich die Leistungsaufnahme der Pumpe, da die Rollen in diesem Augenblick das Schlauchleitungssegment zu okkludieren beginnen. Heben die Rollen hingegen wieder von dem Schlauchleitungssegment ab, verringert sich die Leistungsaufnahme der Pumpe, da die Rollen in diesem Augenblick das Schlauchleitungssegment nicht mehr okkludieren müssen.

Solange die Schlauchleitung ordnungsgemäß im Pumpenbett liegt und die Verdrängerkörper ordnungsgemäß die Schlauchleitung okkludieren, sind unter der Voraussetzung ordnungsgemäßer Rollenlager und Rückstellfedern hinsichtlich der Leistungsaufnahme und des Drucks keine Unterschiede zu erkennen, wenn die einzelnen Rollen auf das Schlauchsegment treffen oder das Schlauchsegment verlassen. Dies ist aber dann nicht der Fall, wenn die Schlauchleitung nicht ordnungsgemäß im Pumpenbett liegt oder Rollenlager oder Rückstellfedern defekt sind.

Ein Indikator für einen nicht ordnungsgemäßen Betrieb der Pumpe ist eine Abweichung der den einzelnen Verdrängerkörpern, beispielsweise Rollen einer Rollenpumpe, zugeordneten Messsignale die größer als ein vorgegebener Betrag ist. Es können auch Mittelwerte der einzelnen Messsignale über ein vorgegebenes Zeitintervall bestimmt werden, um die Mittelwerte der jeweiligen Anteile miteinander vergleichen zu können. Dadurch wird vermieden, dass den Messsignalen überlagerte Störsignale zu einer falschen Auswertung führen können. Grundsätzlich sind aber auch andere statistische Auswertmethoden möglich, um Abweichungen zwischen den Signalen feststellen zu können.

Für das erfindungsgemäße Verfahren und die erfindungsgemäße Vorrichtung ist unerheblich, ob die Leistungsaufnahme oder der Druck direkt gemessen oder eine mit der Leistungsaufnahme oder dem Druck korrelierende Größe bestimmt wird. Die Leistungsaufnahme kann beispielsweise aus dem Produkt der an dem Elektromotor der Blutpumpe anliegenden Spannung und des in den Motor fließenden Stroms berechnet werden. Es genügt aber auch den Strom als eine mit der Leistung korrelierende Größe zu bestimmen, wenn die Spannung als konstant angenommen werden kann.

Weiterhin ist unerheblich, über wie viele Verdrängerkörper die Schlauchpumpe verfügt. Die allgemein üblichen Rollenpumpen weisen in der Regel nur zwei Rollen auf. Daher genügte es, eine Umdrehung der Rollenpumpe um 360° in zwei Zeitbereiche von jeweils 180° aufzuteilen, was einer halben Umdrehung der Rollenpumpe entspricht, um den beiden Rollen jeweils ein Messsignal zuordnen zu können.

Die Zuordnung der einzelnen Messsignale zu den jeweiligen Verdrängerkörpern kann dadurch erfolgen, dass jeweils eine volle Umdrehung in eine der Anzahl der Verdrängerköper entsprechende Zahl von Winkelbereichen aufgeteilt wird.

Bei einer bevorzugten Ausführungsform der Erfindung wird für die Zuordnung der Messsignale zu den Verdrängerkörpern die Position der einzelnen Verdrängerkörper bestimmt, um feststellen zu können, ob sich der Verdrängerkörper in dem jeweiligen Bereich befindet. Die Feststellung der Position der Verdrängerkörper kann auf einfache Weise mit den allgemein bekannten Hallsensoren oder über die Zählung der Umdrehungen der Pumpe unter Berücksichtigung der Anzahl der Verdrängerkörper erfolgen.

Bei einer weiteren bevorzugten Ausführungsform der Erfindung wird aus den Messsignalen, die den einzelnen Verdrängerkörpern zugeordnet werden, jeweils ein dem Gleichanteil und/oder mindestens ein dem Gleichanteil überlagerter Wechselanteil ermittelt, wobei die Gleichanteile und/oder Wechselanteile der Messsignale miteinander verglichen werden, um eine für einen nicht ordnungsgemäßen Betrieb der Pumpe signifikante Abweichung der Anteile der einzelnen Messsignale feststellen zu können. Die Ermittlung des Gleich- und Wechselanteils der den Verdrängerkörpern zugeordneten Messsignale erfolgt vorzugsweise mit einer Fourieranalyse.

Die Überwachung der Leistungsaufnahme einer Schlauchpumpe einer Blutbehandlungsvorrichtung erfordert keinen größeren technischen Aufwand. Auch die Drucküberwachung ist nicht mit einem großen technischen Aufwand verbunden, da Drucksensoren im Allgemeinen ohnehin im extrakorporalen Blutkreislauf einer Blutbehandlungsvorrichtung vorhanden sind. Die Auswertung der Messgrößen zur Bestimmung des nicht ordnungsgemäßen Betriebs der Schlauchpumpe kann mit der Rechen- und Auswerteinheit (Hardware und Software) erfolgen, die zur Steuerung und Überwachung der Blutbehandlung bei den bekannten Blutbehandlungsvorrichtungen bereits vorhanden ist. Dies setzt in der Regel nur eine entsprechende Programmierung voraus. Es ist aber auch möglich, dass die Vorrichtung zum Überwachen der Schlauchpumpe über eine eigene Rechen- und Auswertungseinheit verfügt. Die Blutbehandlungsvorrichtung und die Überwachungsvorrichtung können eine Geräteeinheit bilden oder auch unterschiedliche Baugruppen sein, die unabhängig voneinander betrieben werden können.

Im Folgenden werden Ausführungsbeispiele der Erfindung unter Bezugnahme auf die Zeichnungen näher erläutert.

Es zeigen:
- FIG. 1: in vereinfachter schematischer Darstellung eine erfindungsgemäße extrakorporale Blutbehandlungsvorrichtung, die über eine erfindungsgemäße Vorrichtung zum Überwachen einer peristaltischen Schlauchpumpe der Blutbehandlungsvorrichtung verfügt,
- FIG. 2: eine alternative Ausführungsform der erfindungsgemäßen extrakoporalen Blutbehandlungsvorrichtung mit der erfindungsgemäßen Vorrichtung zum Überwachen der Schlauchpumpe,
- FIG. 3: eine Prinzipdarstellung einer Rollenpumpe.
- FIG. 4A: dass dem ersten Verdrängerkörper der Schlauchpumpe zugeordnete Messsignal bei einem ordnungsgemäßen Betrieb der Schlauchpumpe,
- FIG. 4B: dass dem zweiten Verdrängerkörper zugeordnete Messsignal bei einem ordnungsgemäßen Betrieb,
- FIG. 5A: dass dem ersten Verdrängerkörper der Schlauchpumpe zugeordnete Messsignal bei einem nicht ordnungsgemäßen Betrieb der Schlauchpumpe,
- FIG. 5B: dass dem zweiten Verdrängerkörper zugeordnete Messsignal bei einem nicht ordnungsgemäßen Betrieb,
- FIG. 6A: das erste Messsignal des ersten Verdrängerkörpers vor einer Zerlegung in einen Gleichanteil und einen oder mehrere Wechselanteile (Harmonische) mittels einer Fourieranalyse bei einem nicht ordnungsgemäßen Betrieb der Schlauchpumpe,
- FIG. 6B: das zweite Messsignal des zweiten Verdrängerkörpers vor Zerlegung in Gleich- und Wechseltanteile bei einem nicht ordnungsgemäßen Betrieb und
- FIG. 7: die Gleich- und Wechselanteile (Harmonische) der den beiden Verdrängerkörpern zugeordneten Messsignale nach einer Zerlegung

Die extrakorporale Blutbehandlungsvorrichtung, insbesondere Hämodialysevorrichtung, verfügt über einen Dialysator 1, der durch eine semipermeable Membran 2 in eine Blutkammer 3 und eine Dialysierflüssigkeitskammer 4 unterteilt ist. Von einem Patienten führt ein arterielle Blutleitung 5, in die eine Blutpumpe 6 geschaltet ist, zu einem Einlass der Blutkammer 3, während von einem Auslass der Blutkammer eine venöse Blutleitung 7 über eine Tropfkammer 8 zu dem Patienten führt.

Die frische Dialysierflüssigkeit wird in einer Dialysierflüssigkeitsquelle 9 bereitgestellt. Von der Dialysierflüssigkeitsquelle 9 führt eine Dialysierflüssigkeitszuführleitung 10 zu einem Einlass der Dialysierflüssigkeitskammer 4 des Dialysators 1, während eine Dialysierflüssigkeitabftihrleitung 11 von einem Auslass der Dialysierflüssigkeitskammer 4 zu einem Abfluss 12 führt. In die Dialysierflüssigkeitsabführleitung 11 ist eine Dialysierflüssigkeitspumpe 13 geschaltet.

Bei der Blutpumpe 6 handelt es sich um eine elektrisch betriebene peristaltische Schlauchpumpe, insbesondere eine Rollenpumpe, wobei die arterielle und venöse Blutleitung 5, 7 flexible Schlauchleitungen sind. Die arterielle Schlauchleitung 5 ist in das Pumpenbett der Rollenpumpe 6 eingelegt.

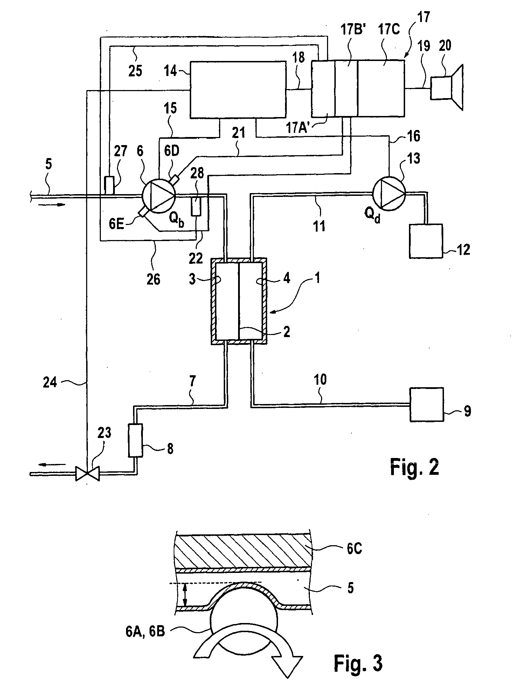

Da Rollenpumpen im Allgemeinen bekannt sind, erübrigt sich eine detaillierte Beschreibung. Fig. 3 zeigt daher nur eine Prinzipdarstellung der Funktionsweise der Rollenpumpe 6, die im vorliegenden Ausführungsbeispiel über zwei Rollen 6A, 6B verfügt, von denen in Fig. 3 nur eine Rolle gezeigt ist. Die Rollen 6A und 6B sind an einem nicht dargestellten Rotor drehbar gelagert. Die Schlauchleitung, im vorliegenden Ausführungsbeispiel die arterielle Blutleitung 5, befindet sich zwischen den Rollen und einem Stator, der eine Rollenbahn 6C als Widerlager bildet. Die Rollenbahn 6C, in dem die arterielle Schlauchleitung 5 liegt, umschließt den mit Rollen besetzten Rotor, wobei die Rollen an dem Rotor gegen die Rollenbahn vorgespannt sind.

Die Dialysevorrichtung verfügt über eine zentrale Steuereinheit 14, die mit der Blutpumpe 6 und der Dialysierflüssigkeitspumpe 13 über Steuerleitungen zu 15, 16 verbunden ist. Die Steuereinheit 14 stellt eine bestimmte Spannung bzw. einen bestimmten Strom zum Betreiben der Blutpumpe 6 und der Dialysierflüssigkeitspumpe 13 zur Verfügung, so dass Blut in den Blutleitungen 5, 7 mit einer vorgegebenen Bluttflussrate Q_{b} und Dialysierflüssigkeit in den Dialysierflüssigkeitsleitungen 10, 11 mit einer vorgegebenen Dialysierflüssigkeitsrate Q_{d} strömen.

Die Dialysevorrichtung kann noch über weitere Komponenten, beispielsweise eine Bilanziereinrichtung oder Ultrafiltrationseinrichtung sowie verschiedene Sensoren zur Überwachung der Blutbehandlung verfügen, die der besseren Übersichtlichkeit halber jedoch nicht dargestellt sind.

Die erfindungsgemäße Vorrichtung zum Überwachen des ordnungsgemäßen Betriebs der Schlauchpumpe wird bei dem vorliegenden Ausführungsbeispiel als Bestandteil der extrakorporalen Blutbehandlungsvorrichtung beschrieben. Bei dem vorliegenden Ausführungsbeispiel wird die Blutpumpe 6 überwacht. Grundsätzlich kann aber auch der ordnungsgemäße Betrieb anderer Pumpen der Blutbehandlungsvorrichtung überwacht werden, beispielsweise einer Substituatpumpe.

Auch wenn die Vorrichtung zum Überwachen der Blutpumpe im vorliegenden Ausführungsbeispiel mit einer extrakorporalen Blutbehandlungsvorrichtung beschrieben ist, kann die Vorrichtung aber auch eine selbstständige Baugruppe bilden, die bei sämtlichen medizin-technischen Einrichtungen zur Überwachung des ordnungsgemäßen Betriebs von peristaltischen Schlauchpumpen eingesetzt werden kann.

Nachfolgend wird die Funktionsweise der Vorrichtung zum Überwachen der Blutpumpe 6 im Einzelnen erläutert.

Die Überwachung der Blutpumpe 6 beruht bei dem Ausführungsbeispiel auf der Auswertung der von der Blutpumpe 6 aufgenommenen Leistung. Die Blutpumpe 6 verfügt über einen Gleichstrom-Elektromotor zum Antrieb des Pumpenrotors. Da die in diesem Fall an dem Elektromotor anliegende Spannung als konstant angenommen werden kann, genügt es, den in den Elektromotor fließenden Strom zu messen. Es sei bemerkt, dass die Blutpumpe grundsätzlich auch mit einem Wechselstrommotor oder anderen Motorantrieben betrieben werden kann.

Die Überwachungsvorrichtung 17 verfügt über nur schematisch dargestellte Mittel 17A zum Bestimmen des Pumpenstroms I(t), die über eine Datenleitung 18 mit der zentralen Steuereinheit 14 der Blutbehandlungsvorrichtung verbunden sind, die für den Betrieb der Blutpumpe 6 einen bestimmten Pumpenstrom einstellt.

Die Überwachungsvorrichtung 17 verfügt weiterhin über Mittel 17B zum Erfassen des Pumpenstroms I_{1,2}(t), der dem ersten oder zweiten Verdrängerkörper, beispielsweise der ersten oder zweiten Rolle 6A und 6B der Rollenpumpe 6 zugeordnet wird. Der Pumpenstrom I_{1,2}(t) der ersten oder zweiten Rolle 6A, 6B stellt ein erstes und zweites Messsignal dar, die miteinander verglichen werden. Hierzu verfügt die Überwachungsvorrichtung 17 über Mittel 17C zum Vergleichen der den beiden Rollen 6A, 6B zugeordneten Messsignale I₁(t) und I₂(t). Auf der Grundlage des Vergleichs beider Messsignale wird auf einen ordnungsgemäßen oder nicht ordnungsgemäßen Betrieb der Pumpe geschlossen.

Bei einem nicht ordnungsgemäßen Betrieb der Pumpe wird ein Steuersignal erzeugt, dass über die Datenleitung 18 an die zentrale Steuereinheit 14 der Blutbehandlungsvorrichtung übertragen wird. Die zentrale Steuereinheit 14 der Blutbehandlungsvorrichtung kann dann einen Eingriff in die Maschinensteuerung vornehmen. Weiterhin kann bei der Feststellung eines nicht ordnungsgemäßen Betriebs der Pumpe ein Alarmsignal erzeugt, dass über eine Datenleitung 19 an eine optische und/ oder akustische Alarmeinheit 20 übertragen wird, die eine optischen und/oder akustischen Alarm gibt.

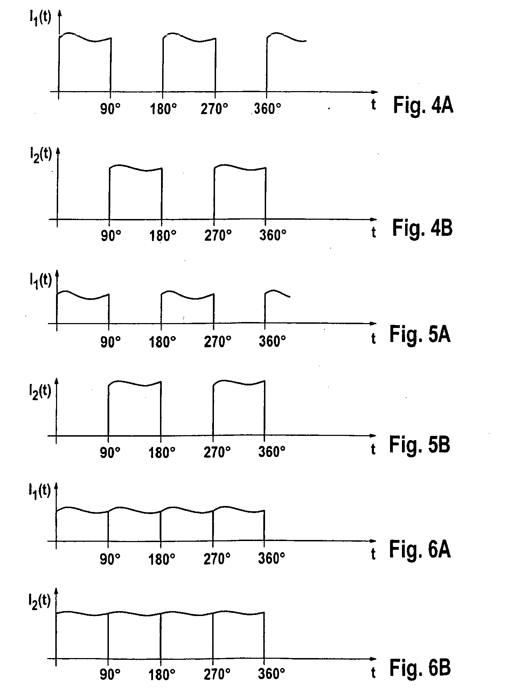

Unter Bezugnahme auf die Figuren 4 bis 6 wird nachfolgend die Zuordnung der Messsignale zu den beiden Verdrängerkörpern 6A und 6B der peristaltischen Schlauchpumpe 6 beschrieben.

Die Umdrehung des Rotors der Rollenpumpe 6 mit den Rollen 6A und 6B wird in einen ersten Winkelbereich von 0-180° und einen zweiten Winkelbereich von 180-360° unterteilt. Der erste Winkelbereich von 0-180° wird der ersten Rolle 6A zugeordnet, während der zweite Winkelbereich von 180-360° der zweiten Rolle 6B zugeordnet wird. Um die Position der Rollen 6Aund 6B des Rotors feststellen zu können, verfügt die Rollenpumpe über einen ersten und einen zweiten Hallsensor 6D und 6E, die jeweils einem der beiden Winkelbereiche zugeordnet sind. Es ist auch möglich, nur einen Hallsensor zu verwenden und die zwischen zwei Signalen des Sensors liegenden Winkelbereiche über die verstrichene Zeit zu interpolieren. Wenn die erste Rolle 6A in den ersten Winkelbereich eintritt, erzeugt der erste Hallsensor 6D ein Signal, während der zweite Hallsensor 6E bei Eintritt der zweiten Rolle 6B in den zweiten Winkelbereich ein Signal erzeugt. Die Signale der Hallsensoren müssen nicht streng geometrisch der Position einer Rolle entsprechen. Vielmehr kann die Zuordnung auch mit einem entsprechenden Offsetwinkel erfolgen. Beide Signale werden von den Mitteln 17B zum Erfassen der den Rollen zugeordneten Ströme über Datenleitungen 21, 22 empfangen. Die Mittel 17B zum Erfassen der jeweiligen Pumpenströme erzeugen ein erstes Signal I₁(t), das den im ersten Winkelbereich 0-180° gemessenen Pumpenstrom entspricht und ein zweites Messsignal I₂(t), das dem im zweiten Winkelbereich 180-360° gemessenen Strom entspricht.

Fig. 4A zeigt den der ersten Rolle 6A zugeordneten Pumpenstrom I₁(t), während Fig. 4B den der zweiten Rolle 6B zugeordneten Pumpenstroms I₂(t) zeigt. Die Figuren 4A und 4B zeigen den Fall des ordnungsgemäßen Betriebs der Blutpumpe. In diesem Fall unterscheiden sich die den beiden Rollen zugeordneten Pumpenströme I₁(t) und I₂(t) nicht wesentlich voneinander, d.h. es sind allenfalls geringfügige Abweichungen zu erkennen.

Die Figuren 5A und 5B zeigen hingegen den Fall eines nicht ordnungsgemäßen Betriebs der Blutpumpe. In diesem Fall unterscheidet sich der Pumpenstrom I₁(t), der der ersten Rolle zugeordnet ist, von dem Pumpenstrom I₂(t), der der zweiten Rolle zugeordnet ist. Der erste Pumpenstrom ist kleiner als der zweite Pumpenstrom. In diesem Fall ist beispielsweise die Schlauchleitung nicht ordnungsgemäß in das Rollenlager eingelegt oder das Rollenlager oder die Rückstellfeder einer der beiden Rollen ist defekt. Der nicht ordnungsgemäße Betrieb wird durch einen Vergleich der beiden Pumpenströme I₁(t) und I₂(t) festgestellt.

Die erfindungsgemäße Vorrichtung sieht zum Vergleich der Pumpenströme eine Fourieranalyse des Pumpenstroms vor. Hierzu wird aus dem ersten Messsignal I₁(t), das nur das erste Winkelfenster umfasst, und aus dem zweiten Messsignal I₂(t), das nur das zweite Winkelfenster umfasst, jeweils ein kontinuierliches Messsignal I₁(t) und I₂(t) erzeugt, das in den Figuren 6A und 6B gezeigt ist. Hierzu kann beispielsweise für den jeweils nicht erfassten Winkelbereich der Pumpenstrom des vorausgehenden oder nachfolgenden Winkelbereichs angenommen werden. Beispielsweise wird das erste Messsignal I₁(t) dadurch gewonnen, dass jeweils für den zweiten Winkelbereich von 180-360° das Messsignal des vorausgehenden Winkelbereichs von 0-180° angenommen wird. Das Messsignal I₂(t), das der zweiten Rolle zugeordnet ist, wird entsprechend erzeugt. Dem Fachmann sind auch andere mathematische Methoden geläufig, um beide Messsignale durch eine Fourieranalyse analysieren zu können.

Daraufhin werden beide Messsignale I₁(t) und I₂(t) mittels einer Fourieranalyse in einen Gleichanteil und die erste Harmonische zerlegt. Das Messsignal kann aber auch in weitere Harmonische zerlegt werden, die der Auswertung zugrunde gelegt werden. Hierfür fügen die Mittel 17C zum Vergleichen der Pumpenströme über Mittel zur Fourieranalyse.

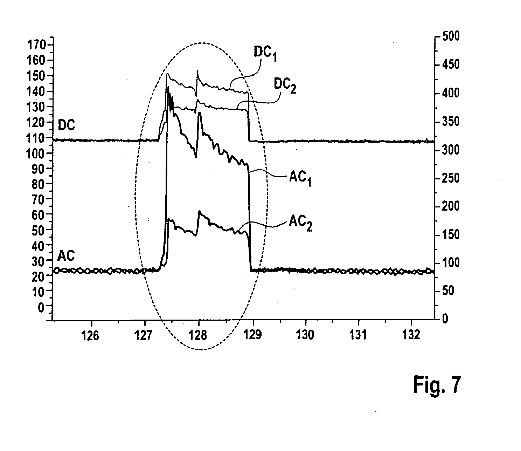

Fig. 7 zeigt das Ergebnis der Fourieranalyse beider Pumpenströme. Während des in Fig. 7 nicht dargestellten Falls eines ordnungsgemäßen Betriebs der Blutpumpe unterscheiden sich die Gleichanteile DC₁ und DC₂ nicht voneinander. Auch die erste Harmonische AC₁ und AC₂ unterscheiden sich während des ordnungsgemäßen Betriebs der Pumpe nicht wesentlich voneinander. Bei dem in Fig. 7 dargestellten Fall eines nicht ordnungsgemäßen Betriebs der Pumpe hingegen weicht der Gleichanteil DC₁ von dem Gleichanteil DC₂ deutlich ab. Auch die erste Harmonische AC₁ weicht deutlich von der zweiten Harmonischen AC₂ ab. Die Mittel 17C zum Vergleichen der Pumpenströme vergleichen zur Feststellung des nicht ordnungsgemäßen Betriebs der Pumpe entweder den Gleichanteil DC₁, der der ersten Rolle zugeordnet ist, mit dem Gleichanteil DC₂, der der zweiten Rolle der Rollenpumpe zugeordnet ist, und/oder die erste Harmonische AC₁ der ersten Rolle mit der ersten Harmonischen AC₂ der zweiten Rolle.

Wenn die Differenz zwischen den beiden Gleichanteilen AC₁ und AC₂ und/oder den beiden Wechselanteilen DC₁ und DC₂ größer als ein vorgegebener Betrag ist, wird auf einen nicht ordnungsgemäßen Betrieb der Pumpe geschlossen, was beispielsweise dann der Fall ist, wenn das Blutschlauchsegment nicht ordnungsgemäß im Pumpenbett liegt oder die Rückstellfeder einer der beiden Rollen defekt ist. Bei dem vorliegenden Ausführungsbeispiel zeigt sich ein nicht ordnungsgemäßer Betrieb der Blutpumpe auf der Zeitskala zwischen ca. 127 bis 129 Minuten. Nach Feststellung des nicht ordnungsgemäßen Betriebs der Blutpumpe wird das Steuersignal für die Steuereinheit 14 und das Alarmsignal für die Alarmeinheit 20 erzeugt, so dass ein Eingriff in die Maschinensteuerung vorgenommen und ein Alarm gegeben wird. Als Eingriff in die Maschinensteuerung kann beispielsweise die Blutpumpe 6 angehalten und eine in der venösen Blutleitung 7 angeordnete Schlauchklemme 23 geschlossen werden, die über eine Steuerleitung 24 mit der Steuereinheit 14 verbunden ist.

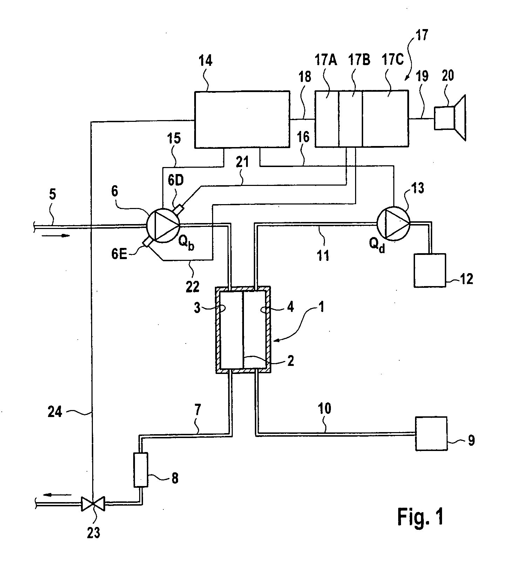

Fig. 2 zeigt eine alternative Ausführungsform der Erfindung, die sich von der unter Bezugnahme auf Fig. 1 beschriebenen Ausführungsform nur dadurch unterscheidet, dass die Auswertung nicht auf der Grundlage der Leistungsaufnahme, sondern des Drucks in der arteriellen Blutleitung stromauf oder stromab der Blutpumpe 6 erfolgt. Daher sind die einander entsprechenden Teile der Blutbehandlungs- und Überwachungsvorrichtung mit den gleichen Bezugszeichen versehen. Die Überwachungsvorrichtung 17 verfügt anstelle der Mittel (17A) zum Bestimmen des Pumpenstroms über Mittel 17A' zum Bestimmen des Drucks in der arteriellen Blutleitung 5 stromauf und stromab der Blutpumpe 6, die über Datenleitungen 25, 26 mit einem Drucksensor 27 stromauf der Blutpumpe 6 bzw. einem Drucksensor 28 stromab der Pumpe verbunden sind. Anstelle der Mittel (17B) zum Erfassen der den einzelnen Verdrängerkörpern zugeordneten Pumpenströme sind Mittel (17B') zum Erfassen der den Verdrängerkörpern zugeordneten Drücke vorgesehen. Obwohl in Fig. 2 beide Drucksensoren dargestellt sind, genügt grundsätzlich eine Druckmessung stromauf oder stromab der Blutpumpe 6.

Die Auswertung der Messsignale erfolgt bei der alternativen Ausführungsform wie bei der ersten Ausführungsform, die eine Auswertung der Leistungsaufnahme der Blutpumpe vorsieht. Die einzige Ausnahme liegt daran, dass anstelle des Pumpenstroms der Druck gemessen und den einzelnen Verdrängerkörpern zugeordnet wird. Daher wird auf die Beschreibung der Ausführungsform von Fig. 1 Bezug genommen..

## Patentansprüche

1. Verfahren zum Überwachen einer peristaltischen Schlauchpumpe zur Förderung einer Flüssigkeit in einer Schlauchleitung, insbesondere einer peristaltischen Schlauchpumpe einer extrakorporalen Blutbehandlungsvorrichtung, wobei die Schlauchpumpe mehrere Verdrängerkörper zum Okkludieren der Schlauchleitung aufweist, wobei
während des Betriebs der Pumpe die von der Pumpe aufgenommene Leistung oder eine mit der Leistung korrelierende Größe und/oder der Druck in dem Schlauchsegment stromauf oder stromab der Pumpe oder eine mit dem Druck stromauf oder stromab der Pumpe korrelierende Größe bestimmt wird,
**dadurch gekennzeichnet,**
**dass** die von der Pumpe aufgenommene Leistung oder eine mit der Leistung korrelierende Größe und/oder
der Druck in dem Schlauchsegment stromauf oder stromab der Pumpe oder eine mit dem Druck stromauf oder stromab der Pumpe korrelierende Größe
für jeweils einen dem jeweiligen Verdrängerkörper zugeordneten Zeitbereich erfasst wird, so dass den einzelnen Verdrängerkörpern zugeordnete Messsignale ermittelt werden, und
**dass** die den einzelnen Verdrängerkörpern zugeordneten Messsignale miteinander verglichen werden, und
**dass** bei einer Abweichung der einzelnen Messsignale, die größer als ein vorgegebener Betrag ist, auf einen nicht ordnungemäßen Betrieb der Pumpe geschlossen wird.

2. Verfahren nach Anspruch 1, **dadurch gekennzeichnet, dass** aus den Messsignalen, die den einzelnen Verdrängerkörpern zugeordnet sind, jeweils ein Gleichanteil und/oder mindestens ein dem Gleichanteil überlagerter Wechselanteil ermittelt wird, wobei die Gleichanteile bzw. Wechselanteile der einzelnen Messsignale miteinander verglichen werden.

3. Verfahren nach Anspruch 1 oder 2, **dadurch gekennzeichnet, dass** in einem vorgegebenen Zeitintervall ein Mittelwert der den einzelnen Verdrängerkörpern zugeordneten Gleichanteile und/oder Wechselanteile bestimmt wird, und dass die Mittelwerte der jeweiligen Gleichanteile bzw. Wechselanteile miteinander verglichen werden, wobei bei einer Abweichung der Mittelwerte, die größer als ein vorgegebener Betrag ist, auf einen nicht ordnungsgemäßen Betrieb der Schlauchpumpe geschlossen wird.

4. Verfahren nach einem der Ansprüche 1 bis 3, **dadurch gekennzeichnet, dass** zur Bestimmung des Gleich- und/oder Wechselanteils der einzelnen Messsignale eine Fourierananalyse der Messsignale durchgeführt wird.

5. Verfahren nach einem der Ansprüche 1 bis 4, **dadurch gekennzeichnet, dass** für die Zuordnung der Messsignale zu den einzelnen Verdrängerkörpern die Position des Verdrängerkörpers bestimmt wird.

6. Verfahren nach einem der Ansprüche 1 bis 5, **dadurch gekennzeichnet, dass** die Schlauchpumpe eine okklusive Rollenpumpe ist, wobei die Schlauchleitung zwischen einem Stator, der eine Rollenbahn als Widerlager bildet, und einem mit drehbar gelagerten Rollen besetzten Rotor angeordnet ist.

7. Verfahren nach einem der Ansprüche 1 bis 6, **dadurch gekennzeichnet, dass** eine peristaltische Schlauchpumpe einer extrakorporalen Blutbehandlungsvorrichtung
betrieben wird.

8. Verfahren nach Anspruch 7, **dadurch gekennzeichnet, dass** die peristaltische Schlauchpumpe in einer arteriellen Schlauchleitung angeordnet ist, die zu einer Blutbehandlungseinheit führt, von der eine venöse Schlauchleitung abgeht, wobei die arterielle und venöse Schlauchleitung zusammen mit der Blutbehandlungseinheit einen extrakorporalen Blutkreislauf bilden.

9. Vorrichtung zum Überwachen einer peristaltischen Schlauchpumpe zur Förderung einer Flüssigkeit in einer Schlauchleitung, wobei die Schlauchpumpe mehrere Verdrängerkörper zum Okkludieren der Schlauchleitung aufweist, mit
Mitteln (17B, 17B') zum Erfassen der während des Betriebs der Pumpe von der Pumpe aufgenommenen Leistung oder einer mit der Leistung korrelierende Größe und/oder des Drucks in dem Schlauchsegment stromauf oder stromab der Pumpe oder einer mit dem Druck stromauf oder stromab der Pumpe korrelierende Größe,
**dadurch gekennzeichnet,**
**dass** die Mittel (17B, 17B') zum Erfassen der von der Pumpe aufgenommenen Leistung oder der mit der Leistung korrelierenden Größe und/oder des Druck in dem Schlauchsegment stromauf oder stromab der Pumpe oder einer mit dem Druck stromauf oder stromab der Pumpe korrelierende Größe derart ausgebildet sind, dass für jeweils einen dem jeweiligen Verdrängerkörper zugeordneten Zeitbereich den einzelnen Verdrängerkörpern zugeordnete Messsignale ermittelt werden und
**dass** die Vorrichtung aufweist:
Mittel (17C) zum Vergleichen der den einzelnen Verdrängerkörpern zugeordneten Messsignale miteinander, die derart ausgebildet sind, dass bei einer Abweichung der einzelnen Messsignale, die größer als ein vorgegebener Betrag ist, auf einen nicht ordnungemäßen Betrieb der Pumpe geschlossen wird.

10. Vorrichtung nach Anspruch 9, **dadurch gekennzeichnet, dass** die Mittel (17C) zum Vergleichen der Messsignale derart ausgebildet sind, dass aus den Messsignalen, die den einzelnen Verdrängerkörpern zugeordnet sind, jeweils ein Gleichanteil und/oder mindestens ein dem Gleichanteil überlagerter Wechselanteil ermittelt wird, wobei die Gleichanteile bzw. Wechselanteile der einzelnen Messsignale miteinander verglichen werden.

11. Vorrichtung nach einem der Ansprüche 9 bis 10, **dadurch gekennzeichnet, dass** die Mittel (17C) zum Vergleichen der Messsignale Mittel zur Durchführung einer Fourieranalyse der Messsignale zur Bestimmung des Gleich- und/oder Wechselanteils der einzelnen Messsignale aufweisen.

12. Vorrichtung nach einem der Ansprüche 9 bis 11, **dadurch gekennzeichnet, dass** die Schlauchpumpe eine okklusive Rollenpumpe (6) ist, wobei die Schlauchleitung zwischen einem Stator, der eine Rollenbahn (6C) als Widerlager bildet, und einem mit drehbar gelagerten Rollen (6A, 6B) besetzten Rotor angeordnet ist.

13. Vorrichtung nach einem der Ansprüche 9 bis 12, **dadurch gekennzeichnet, dass** die Mittel (17B, 17B') zum Erfassen der von der Pumpe aufgenommenen Leistung und/oder des Drucks Mittel (6E, 6D) zum Bestimmen der Position des Verdrängerkörpers für die Zuordnung der Messsignale zu den einzelnen Verdrängerkörpern aufweisen.

14. Vorrichtung nach einem der Ansprüche 9 bis 13, **dadurch gekennzeichnet, dass** die peristaltische Schlauchpumpe die peristaltische Schlauchpumpe (6) einer extrakorporalen Blutbehandlungsvorrichtung ist.

15. Blutbehandlungsvorrichtung, **dadurch gekennzeichnet, dass** die Blutbehandlungsvorrichtung eine Vorrichtung nach einen der Ansprüche 9 bis 15 aufweist.

16. Blutbehandlungsvorrichtung nach Anspruch 15, **dadurch gekennzeichnet, dass** die Blutbehandlungsvorrichtung eine Blutbehandlungseinheit (1) aufweist, zu der eine arterielle Schlauchleitung (5) führt und von der eine venöse Schlauchleitung (7) abgeht, wobei die peristaltische Schlauchpumpe (6) in der arteriellen Schlauchleitung (5) angeordnet ist.

## Claims

1. Method for monitoring a peristaltic hose pump to convey a fluid in a hose line, in particular a peristaltic hose pump forming part of an extra-corporeal blood treatment apparatus, wherein the hose pump comprises a number of displacement elements for occluding the hose line, whereby During the operation of the pump, the power consumed by the pump or alternatively a value that correlates to the power and/or the pressure in the hose segment upstream or downstream of the pump, is calculated,
**characterized in that**
the power absorbed by the pump or a value that correlates to the power and/or the pressure in the hose segment is determined upstream or downstream of the pump, or to a value that correlates with the pressure upstream or downstream of the pump respectively is calculated for each time range allocated to each displacer element, so that measuring signals allocated to the individual displacer elements are determined, and
that the measuring signals allocated to the individual displacement elements are compared with each other and
that in the event of any deviation of the individual measuring signals exceeding a predetermined amount, it can be concluded that the pump is not functioning properly.

2. A method according to claim 1, **characterized in that** from the measurement signals, allocated to the individual displacement elements DC components and/or at least one AC component superimposed over the DC component is determined and the DC and AC components of the individual measuring signals are compared.

3. A method according to Claim 1 or 2 above, **characterized in that** at a predetermined time interval a mean value is calculated of the individual displacement elements allocated to the DC components and / or AC components, and that the mean values of the respective DC components and AC components are compared with each other, whereby any deviation of the mean values which exceeds a predetermined amount, indicates that the hose pump is not functioning properly.

4. A method according to any one of the preceding Claims, **characterized in that** in order to determine the DC and / or AC component of the individual measurement signals, a Fourier analysis is carried out on the measurement signals.

5. A method according to any one of the preceding Claims 1 to 4, **characterized in that**, for the allocation of the measurement signals to the individual displacement elements, the position of the respective displacement element is determined.

6. A method according to any one of the preceding Claims 1 to 5, **characterized in that** the hose pump is an occlusive roller pump, whereby the hose line is arranged between a stator, which forms a roller path as a counter-bearing, and a rotor fitted with rotatable rollers.

7. A method according to any one of the Claims 1 to 6 above, **characterized in that** a peristaltic hose pump of an extra-corporeal blood treatment apparatus is operated.

8. A method according to Claim 7 above, **characterized in that** the peristaltic hose pump is arranged in an arterial hose line, which leads to a blood treatment unit and issues from a venous hose line, wherein the arterial and venous hose line together form an extra-corporeal blood circuit with the blood treatment unit.

9. An apparatus for monitoring a peristaltic hose pump for conveying a fluid in a hose line, wherein the peristaltic pump includes a number of displacement elements for occluding the hose line, with
Means (17B, 17B') for detecting the power absorbed by the pump during operation, or a value that correlates to the power and / or the pressure in the tubing segment upstream or downstream of the pump or upstream or downstream of the value correlating to the pressure,
**characterized in**
**that** the means (17B, 17B ') for detecting the power absorbed by the pump, or a value that correlates to the power and / or the pressure in the tube segment upstream or downstream of the pump or a value correlating to the pressure upstream or downstream of the pump are formed in such a way that measuring signals allocated to the individual displacement elements are calculated for each time range allocated to the respective displacement elements and
**that** the apparatus comprises:
Means (17C) for comparing the measuring signals allocated to the individual displacement elements with each other, whereby these signals are configured in such a manner that, in the event of any deviation of the individual measurement signals from a predetermined amount, it is concluded that the pump is not operating correctly.

10. Apparatus according to Claim 9 above, **characterized in that** the means (17C) are designed to compare the measurement signals in such a way that from the measurement signals, which are allocated to the individual displacement elements, respectively a DC component and / or at least one AC component superimposed on the DC component is determined, wherein the DC components and AC components of the individual measured signals are compared.

11. Apparatus according to either of the preceding Claims 9 to 10, **characterized in that** the means (17C) for comparing the measurement signals comprise means for carrying out a Fourier analysis of the measured signals to determine the DC and/or the AC component of the individual measurement signals.

12. Apparatus according to any of the preceding Claims 9 to 11, **characterized in that** the hose pump is an occlusive roller pump (6), whereby the hose line is arranged between a stator, which forms a roller conveyor (Fig. 6C) as a counter-bearing and a rotor equipped with rotating rollers (6A, 6B).

13. Apparatus according to any of the preceding Claims 9 to 12, **characterized in that** the means (17B, 17B') for detecting the power absorbed by the pump and/or the pressure comprises means (6E, 6D) for determining the position of the displacement element for the allocation of the measuring signals to the individual displacement elements.

14. Apparatus according to any of the preceding Claims 9 to 13, **characterized in that** the peristaltic hose pump is the peristaltic pump (6) of an extra-corporeal blood treatment apparatus.

15. Blood treatment apparatus, **characterized in that** the blood treatment apparatus comprises a device according to any one of the preceding claims 9 to 15.

16. Blood treatment apparatus according to Claim 15 above, **characterized in that** the blood treatment device comprises a blood treatment unit (1), to which an arterial hose line (5) leads and from which a venous hose line issues (7), wherein the peristaltic hose pump (6) is arranged in the arterial hose line (5).

## Revendications

1. Procédé de surveillance d'une pompe péristaltique servant à refouler un liquide dans un tuyau flexible, en particulier une pompe péristaltique d'un dispositif de traitement du sang extracorporel, la pompe présentant plusieurs corps de refoulement servant à obstruer le tuyau flexible, dans lequel
lors du fonctionnement de la pompe, la puissance absorbée par la pompe ou une grandeur mise en corrélation avec la puissance et/ou
la pression dans le tronçon de tuyau en amont ou en aval de la pompe ou une grandeur mise en corrélation avec la pression en amont ou en aval de la pompe est déterminée, **caractérisé en ce**
**que** la puissance absorbée par la pompe ou une grandeur mise en corrélation avec la puissance et/ou
la pression dans le tronçon de tuyau en amont ou en aval de la pompe ou une grandeur mise en corrélation avec la pression en amont ou en aval de la pompe est détectée pour respectivement une plage de temps associée à chacun des corps de refoulement de sorte que des signaux de mesure associés à chacun des corps de refoulement sont déterminés, et
en ce que les signaux de mesure associés à chacun des corps de refoulement sont comparés les uns aux autres, et
**qu'**en cas d'écart entre les signaux de mesure individuels, supérieur à une valeur prédéfinie, on conclut à un mauvais fonctionnement de la pompe.

2. Procédé selon la revendication 1, **caractérisé en ce qu'**on détermine à partir des signaux de mesure, qui sont associés à chacun des corps de refoulement, respectivement une composante continue et/ou au moins une composante alternative superposée à la composante continue, les composantes continues ou les composantes alternatives des signaux de mesure individuels étant comparées les unes aux autres.

3. Procédé selon la revendication 1 ou 2, **caractérisé en ce qu'**une valeur moyenne des composantes continues et/ou des composantes alternatives associées à chacun des corps de refoulement est déterminée dans un intervalle de temps prédéfini, et **en ce que** les valeurs moyennes des composantes continues ou alternatives respectives sont comparées les unes aux autres, et dans lequel, en cas d'écart entre les valeurs moyennes, supérieur à une valeur prédéfinie, on conclut à un mauvais fonctionnement de la pompe.

4. Procédé selon l'une quelconque des revendications 1 à 3, **caractérisé en ce qu'**on effectue une analyse de Fourier des signaux de mesure aux fins de la détermination des composantes continues et/ou alternatives des signaux de mesure individuels.

5. Procédé selon l'une quelconque des revendications 1 à 4, **caractérisé en ce que** la position du corps de refoulement est déterminée pour l'association des signaux de mesure à chacun des corps de refoulement.

6. Procédé selon l'une quelconque des revendications 1 à 5, **caractérisé en ce que** la pompe est une pompe à galets occlusive, le tuyau flexible étant disposé entre un stator formant une piste de roulement en tant que butée et un rotor pourvu de galets agencés de manière à pouvoir tourner.

7. Procédé selon l'une quelconque des revendications 1 à 6, **caractérisé en ce qu'**une pompe péristaltique d'un dispositif de traitement du sang extracorporel est exploitée.

8. Procédé selon la revendication 7, **caractérisé en ce que** la pompe péristaltique est disposée dans un tuyau flexible artériel, qui mène à une unité de traitement du sang, de laquelle part un tuyau flexible veineux, le tuyau flexible artériel et le tuyau flexible veineux formant conjointement avec l'unité de traitement du sang un système de circulation extracorporelle du sang.

9. Dispositif de surveillance d'une pompe péristaltique servant à refouler un liquide dans un tuyau flexible, la pompe présentant plusieurs corps de refoulement servant à obstruer le tuyau flexible, comprenant
des moyens (17B, 17B') servant à détecter la puissance absorbée par la pompe lors du fonctionnement de cette dernière ou une grandeur mise en corrélation avec la puissance et/ou la pression dans le tronçon de tuyau en amont ou en aval de la pompe ou une grandeur mise en corrélation avec la pression en amont ou en aval de la pompe,
**caractérisé en ce**
**que** les moyens (17B, 17B') servant à détecter la puissance absorbée par la pompe ou la grandeur mise en corrélation avec la puissance et/ou la pression dans le tronçon de tuyau en amont ou en aval de la pompe ou une grandeur mise en corrélation avec la pression en amont ou en aval de la pompe sont réalisés de telle manière que des signaux de mesure associés à chacun des corps de refoulement sont déterminés pour respectivement une plage de temps associée à respectivement un corps de refoulement, et
en ce que le dispositif présente :
des moyens (17C) servant à comparer entre eux les signaux de mesure associés à chacun des corps de refoulement, lesquels sont réalisés de telle manière qu'en cas d'écart entre les signaux de mesure individuels, supérieur à une valeur prédéfinie, on conclut à un mauvais fonctionnement de la pompe.

10. Dispositif selon la revendication 9, **caractérisé en ce que** les moyens (17C) servant à comparer les signaux de mesure sont réalisés de telle manière qu'on détermine à partir des signaux de mesure associés à chacun des corps de refoulement respectivement une composante continue et/ou au moins une composante alternative superposée à la composante continue, les composantes continues ou alternatives des signaux de mesure individuels étant comparées les unes aux autres.

11. Dispositif selon l'une quelconque des revendications 9 à 10, **caractérisé en ce que** les moyens (17C) servant à comparer les signaux de mesure présentent des moyens servant à mettre en oeuvre une analyse de Fourier des signaux de mesure aux fins de la détermination de la composante continue et/ou alternative des signaux de mesure individuels.

12. Dispositif selon l'une quelconque des revendications 9 à 11, **caractérisé en ce que** la pompe est une pompe à galets occlusive (6), le tuyau flexible étant disposé entre un stator formant une piste de roulement en tant que butée (6C) et un rotor pourvu de galets (6A, 6B) agencés de manière à pouvoir tourner.

13. Dispositif selon l'une quelconque des revendications 9 à 12, **caractérisé en ce que** les moyens (17B, 17B') servant à détecter la puissance absorbée par la pompe et/ou la pression présentent des moyens (6E, 6D) servant à déterminer la position du corps de refoulement pour l'association des signaux de mesure à chacun des corps de refoulement.

14. Dispositif selon l'une quelconque des revendications 9 à 13, **caractérisé en ce que** la pompe péristaltique est la pompe péristaltique (6) d'un dispositif de traitement du sang extracorporel.

15. Dispositif de traitement du sang, **caractérisé en ce que** ledit dispositif de traitement du sang présente un dispositif selon l'une quelconque des revendications 9 à 15.

16. Dispositif de traitement du sang selon la revendication 15, **caractérisé en ce que** le dispositif de traitement du sang présente une unité de traitement du sang (1), à laquelle mène un tuyau flexible artériel (5) et de laquelle part un tuyau flexible veineux (7), la pompe péristaltique (6) étant disposée dans le tuyau flexible artériel (5).
